(19) Europäisches Patentamt
European Patent Office
Office européen des brevets

(11) EP 0 921 186 B1

(12) **FASCICULE DE BREVET EUROPEEN**

(45) Date de publication et mention de la délivrance du brevet:
**03.11.2004 Bulletin 2004/45**

(51) Int Cl.7: **C11B 9/00**, C07C 69/716, C11D 3/50, A61L 9/01

(21) Numéro de dépôt: **98122811.7**

(22) Date de dépôt: **01.12.1998**

(54) **Utilisation du 3-méthyl-2-oxopentanoate d'éthyle en tant qu'ingrédient parfumant**

Verwendung des Ethyl 3-methyl-2-oxopnetanoate als Riechstoff

Use of ethyl 3-methyl-2-oxopentanoate as a perfuming ingredient

(84) Etats contractants désignés:
**CH DE ES FR GB IT LI NL**

(30) Priorité: **08.12.1997 CH 282597**

(43) Date de publication de la demande:
**09.06.1999 Bulletin 1999/23**

(73) Titulaire: **FIRMENICH SA**
**1211 Genève 8 (CH)**

(72) Inventeurs:
- **Snowden, Roger L.**
  **74580 Viry (FR)**
- **Pamingle, Hervé**
  **1290 Versoix (CH)**
- **Vial, Christian**
  **1202 Geneve (CH)**

(74) Mandataire: **Salvaterra-Garcia, Maria de Lurdes**
**Firmenich SA**
**Département des Brevets**
**Case Postale 239**
**1211 Genève 8 (CH)**

(56) Documents cités:
**EP-A- 0 838 215** **WO-A-96/10927**

- **DATABASE WPI Week 199345 Derwent Publications Ltd., London, GB; AN 1993-354005 XP002131632 & JP 05 255189 A (HONSHU CHEM. IND.), 5 octobre 1993 (1993-10-05)**
- **Y. AKIYAMA ET AL.: "Reaction of organocadmium reagents with ethyl cyanoformate: preparation of alpha-Keto esters" CHEMISTRY LETTERS., vol. 8, 1983, pages 1231-1232, XP002131630 CHEMICAL SOCIETY OF JAPAN. TOKYO., JP ISSN: 0366-7022**
- **R. KOZLOWSKI ET AL.: "Lower aliphatic 2-oxoacids and their ethyl esters from ethyl esters of 2-hydroxy acids" ORGANIC PREPARATIONS AND PROCEDURES INTERNATIONAL., vol. 21, no. 1, 1989, pages 75-82, XP002131631 ORGANIC PREPARATION AND PROCEDURES CO., NEWTON HIGHLANDS, MA., US ISSN: 0030-4948**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 921 186 B1

Printed by Jouve, 75001 PARIS (FR)

## Description

La présente demande a trait au domaine de la parfumerie. Elle concerne, plus particulièrement, l'utilisation du 3-méthyl-2-oxopentanoate d'éthyle obéissant à la formule

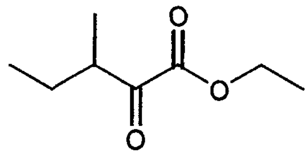

sous forme d'un isomère optiquement actif ou d'un mélange d'isomères, en tant qu'ingrédient parfumant.

Ledit ester sous forme de mélange racémique est un composé dont la structure est connue en soi. En effet, dans la littérature, il existe plusieurs références qui divulguent ce composé, soit en le mentionnant seulement en tant que molécule, soit en décrivant en détail sa préparation. On peut citer ainsi les références suivantes : R. Kozlowski et al., Org. Prep. Proced. Int., 21(1), 75-82 (1989) ; Y. Akiyama et al., Chem. Pharm. Bull. 32(5), 1800-1807 (1984); Y. Akiyama et al., Chem. Lett. 8, 1231-1232 (1983); P. Yates et al., Can. J. Chem. 61(7), 1397-1404 (1983); H.M. Walborsky et al., J. Org. Chem. 39(5), 604-607 (1974); L.N. Akimova et al., Zh. Org. Khim. 5(9), 1569-1571 (1969). On ne trouve, cependant, dans toutes ces références, ni une description de l'odeur de l'ester de la présente invention, ni une suggestion de son utilisation en parfumerie.

En plus, on connaît l'utilisation des acides de structure

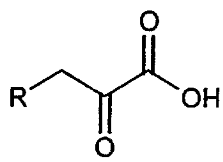

dans laquelle R peut représenter l'hydrogène ou une grande variété de radicaux alkyles, saturés ou insaturés, linéaires ou ramifiés, parmi eux l'acide 3-méthyl-2-oxopentanoique, utilisés dans le domaine des arômes. La demande internationale WO 96/10927 décrit l'utilisation de ladite classe de composés dans des aliments de nature très variée, auxquels ils impartissent un impact accru dans la bouche. On mentionne, d'une façon générale, également les esters alkyliques de $C_1$ à $C_4$ desdits acides en tant qu'ingrédients aromatisants utiles, sans toutefois donner un exemple concret de préparation ou même d'application de ces esters alkyliques. Encore une fois, on ne trouve aucune description de la qualité olfactive des esters de la présente invention.

D'une façon surprenante, nous avons maintenant découvert que le 3-méthyl-2-oxopentanoate d'éthyle ou l'un de ses isomères sont des ingrédients parfumants très utiles, ayant une odeur jusqu'à présent inconnue et inhabituelle, réunissant des notes fraîches-fruitées avec une note de type noix.

La présente invention a donc trait à l'utilisation en tant qu'ingrédient parfumant du 3-méthyl-2-oxopentanoate d'éthyle ou de l'un de ses isomères.

Plus précisément, les composés de l'invention présentent une note complexe de type noix, qui peut être décrite comme rappelant l'odeur de la noix fraîche mélangée à celle de brou de noix. Cette note est étonnamment fruitée; on trouve de plus une connotation de noisette. La note caractéristique de noix-noisettes fraîches est accompagnée d'une odeur rappelant le rhum. D'une façon générale, l'odeur est très naturelle, excellente et peu commune.

Le 3-méthyl-2-oxopentanoate d'éthyle apporte une nouvelle note à la palette du parfumeur, une note fruitée noix très naturelle qui n'était pas disponible jusqu'à présent. Cette combinaison inhabituelle de la connotation fruitée avec une odeur noix-rhum très naturelle, confère une grande valeur au composé de l'invention pour utilisation dans la parfumerie.

Par ailleurs, nous avons pu évaluer les propriétés organoleptiques des isomères optiquement actifs du 3-méthyl-2-oxopentanoate d'éthyle: alors que le (+)-(S)-3-méthyl-2-oxopentanoate d'éthyle présente une note de type noix, accompagnée d'une odeur de brou de noix, piquante, éthérée et légèrement fruitée, le (-)-(R)-3-méthyl-2-oxopentanoate d'éthyle présente également une note de type noix, mais son odeur est plus piquante, plus sèche, moins puissante et ne possède pas de note fruitée de type pomme.

Les composés de l'invention se prêtent surtout à une utilisation en parfumerie fine, à savoir dans les parfums, les eaux de toilette ou les lotions après-rasage.

Bien entendu, leur utilisation n'est cependant pas limitée aux produits mentionnés auparavant, et ces composés se prêtent également à tous les autres emplois courants en parfumerie, à savoir au parfumage de savons et gels de douche ou de bain, de produits d'hygiène ou de traitement des cheveux comme les shampoings, ainsi que de déodorants corporels et désodorisants ambiants ou encore de préparations cosmétiques.

Les composés peuvent également être employés dans des applications telles que détergents liquides ou solides destinés au traitement de textiles, adoucissants textiles, ou encore des compositions détergentes ou produits d'entretien destinés au nettoyage de la vaisselle ou de surfaces variées, qu'ils soient voués à un usage domestique ou industriel.

Dans ces applications, ils peuvent être utilisés seuls ou en mélange avec d'autres ingrédients parfumants, des solvants ou adjuvants d'usage courant en parfumerie. La nature et variété de ces coingrédients n'appelle pas une description plus détaillée ici, qui ne saurait d'ailleurs être exhaustive, l'homme de l'art étant à même de les choisir de par ses connaissances générales et en fonction de la nature du produit à parfumer et de l'effet olfactif recherché. Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les produits divers susmentionnés varient dans une gamme de valeurs étendue. Ces valeurs sont dépendantes de la nature de l'article ou produit que l'on veut parfumer et de l'effet olfactif recherché, ainsi que de la nature des coingrédients dans une composition donnée lorsque les composés de l'invention sont utilisés en mélange avec des coingrédients parfumants, des solvants ou des adjuvants d'usage courant dans l'art.

A titre d'exemple, on peut citer des concentrations typiques de l'ordre de 0,1 à 10% voir plus, en poids de ces composés, par rapport au poids de composition parfumante dans laquelle ils sont incorporés. Des concentrations bien inférieures à celles-ci peuvent être utilisées lorsque ces composés sont directement appliqués dans le parfumage des produits de consommation divers cités auparavant.

L'invention a également trait à un procédé de préparation de chacun des esters optiquement actifs de l'invention, à partir des hydroxyesters correspondants, à savoir le (+)-(2S, 3S)-2-hydroxy-3-méthylpentanoate d'éthyle et le (+)-(2S, 3R)-2-hydroxy-3-méthylpentanoate d'éthyle. Les deux réactions ont été effectuées de manière similaire, en ajoutant successivement de l'acétate de sodium et du chlorochromate de pyridine à une solution de (+)-(2S, 3S)-2-hydroxy-3-méthylpentanoate d'éthyle, respectivement de (+)-(2S, 3R)-2-hydroxy-3-méthylpentanoate d'éthyle. Ces synthèses ainsi que la préparation des hydroxyesters seront décrites de façon plus détaillée dans les exemples ci-dessous.

L'invention sera maintenant décrite de façon plus détaillée dans les exemples suivants, dans lesquels les températures sont indiquées en degrés Celsius et les abréviations ont le sens usuel dans l'art.

Exemple 1

Préparation des isomères optiquement actifs selon l'invention

**1. Synthèse du (+)-(S)-3-méthyl-2-oxopentanoate d'éthyle**

La synthèse a été effectuée en trois étapes selon le schéma suivant :

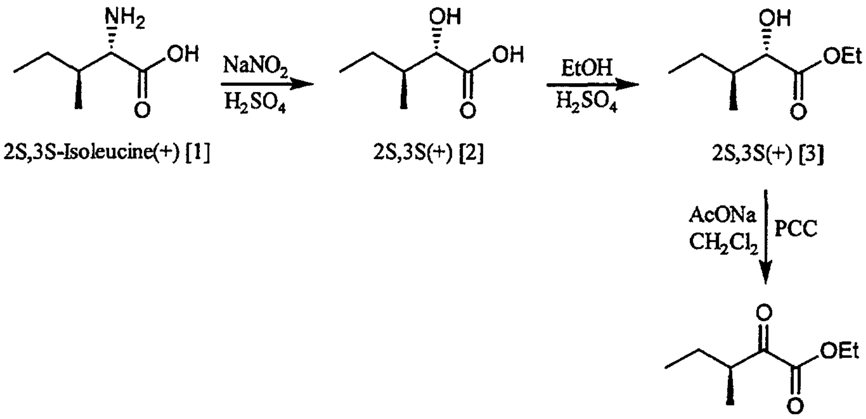


2S,3S-Isoleucine(+) [1]    2S,3S(+) [2]    2S,3S(+) [3]

[4]

*1.1. Synthèse de l'acide (+)-(2S, 3S)-2-hydroxy-3-méthylpentanoique [2]*

On a ajouté lentement (ca. 12h) à une solution de L-isoleucine (+) [1] (11,73 g, 89,4 mmole, $[\alpha]^D{}_{20}$= +40° (5% dans HCl 6M)) dans 300 ml de $H_2SO_4$ aq. 1N à 2°, une solution de $NaNO_2$ (19,5 g, 0,28 mole) dans 300 ml d'eau déminéralisée. Après agitation durant la nuit à température ambiante, on a ajouté 7.7 g de $NaHCO_3$ en poudre pour atteindre un pH supérieur à 2. On a saturé la solution avec du NaCl et extrait 6 fois à l'aide d'acétate d'éthyle, en maintenant le pH entre 2 et 3 ($H_2SO_4$ aq. 1N, pH mètre). Après séchage ($Na_2SO_4$), on a concentré la couche organique et recristallisé le produit brut (10,73 g, 91%) au moyen d'hexane/acétate d'éthyle 9/1 à -30° pour obtenir l'acide (+) -(2S, 3S)-2-hydroxy-3-méthylpentanoique pure [2].

Données analytiques :

p.f. = 52-54°
$[\alpha]^D{}_{20}$ = +22,4° (1,25% dans $CHCl_3$)
SM : 132($M^+$,0) : m/e : 87(28), 76(100), 69(12), 57(25), 45(23), 29(14)
$^1$H-RMN($CDCl_3$) : 0,90(t, J=7, 3H); 0,98(d, J=7, 3H) ; 1,26(m, 1H) ; 1,41(m, 1H) ; 1,83(m, 1H) ; 4,13(d, $J_1$=4)
$^{13}$C-RMN($CDCl_3$) : 2q : 11,7, 15,2 ; 1t : 23,8 ; 2d : 38,7, 74,7 ; 1s : 177,9

*1.2. Synthèse du (+)-(2S, 3S)-2-hydroxy-3-méthylpentanoate d'éthyle [3]*

On a ajouté à une solution de (+)-hydroxy acide [2] (4,57 g, 34,6 mmole) dans 46 ml d'éthanol absolu, 0,3 g de $H_2SO_4$ conc. et on a chauffé le mélange à 60° durant 5 h. Après un traitement usuel et concentration, on a distillé le composé (four à bulles, 110-120°, 18 mbars) pour obtenir 4,65 g (rendement: 84%) de (+)-hydroxy ester, avec une pureté de 95,3% [3]. On a purifié un échantillon par chromatographie flash sur $SiO_2$ (heptane/ether 65/35).
On a trouvé, par analyse CG sur colonne chirale (CHIRASIL-DEX CB, 25 m x 0,25 mm, isoth. 90°), que le produit était énantiomériquement pur (il contenait 2,5% de 2R, 3S-diastéréoisomère.

Données analytiques :

$[\alpha]^D{}_{20}$= + 16,7° (1,75% dans $CHCl_3$)
SM : 160($M^+$,1) : m/e : 104(47), 87(95), 76(86), 69(38), 57(32), 45(100), 29(74)
$^1$H-RMN($CDCl_3$) : 0,90(t, J=7, 3H) ; 0,99(d, J=7, 3H) ; 1,30(t, J=7, 3H) ; 1,19-1,43(m, 2H) ; 1,81(m, 1H) ; 2,74(d, J=6, 1H, disparition par addition de $D_2O$) ; 4,07(dd, $J_1$=4,$J_2$=6, 1H, déformation à d, J=4 par addition de $D_2O$) ; 4,26(m, 2H)
$^{13}$C-RMN($CDCl_3$) : 3q : 11,8, 14,2, 15,4 ; 2t : 23,8, 61,5 ; 2d : 39,1, 74,6 ; 1s : 175,0

*1.3. Synthèse du (+)-(S)-3-méthyl-2-oxopentanoate d'éthyle [4]*

On a ajouté successivement à une solution de (+)-hydroxy ester [3] (2,0 g, 12,5 mmole) dans 20 ml de $CH_2Cl_2$ à température ambiante, de l'acétate de sodium (312 mg, 3,8 mmole) et du PCC (chlorochromate de pyridine, 4,04 g, 18,75 mmole). Après 24h à température ambiante, la conversion était seulement de 60% et on a rajouté 2,02 g de PCC. On a maintenu le mélange sous agitation durant 96h à température ambiante, rapidement filtré sur $SiO_2$, concentré et purifié par chromatographie flash sur $SiO_2$ pour obtenir 1,58 g d'ester [4]. A ce stade, un contrôle CG sur colonne chirale (CHIRASIL-DEX CB, 25 m x 0,25 mm, isoth. 80°) a montré une pureté de 100% et un ee (excès énantiomérique) de 100%. Après distillation sur four à bulles (Eb=100°, 16 mbars), on a obtenu 1,51 g (rendement: 76,3%) de (+)-ester [4], pure.

Données analytiques:

$[\alpha]^D_{20}$= + 38,4° (0,7% dans $CHCl_3$)
SM : 158($M^+$,6) : m/e : 102(4), 85(60), 69(3), 57(100), 41(25), 29(21)
$^1$H-RMN($CDCl_3$) : 0,92(t, J=7, 3H) ; 1,13(d, J=7, 3H) ; 1,38(t, J=7, 3H) ; 1,45(m, 1H) ; 1,77(m, 1H) ; 3,14(m, 1H) ; 4,32(q, J=7, 2H)
$^{13}$C-RMN($CDCl_3$) : 3q : 11,4, 14,1, 14,6 ; 2t : 25,0, 62,2 ; 1d : 43,6 ; 2s : 162,1, 198,3

**2. Synthèse du (-)-(R)-3-méthyl-2-oxopentanoate d'éthyle**

La synthèse a été effectuée en trois étapes, selon le schéma suivant:

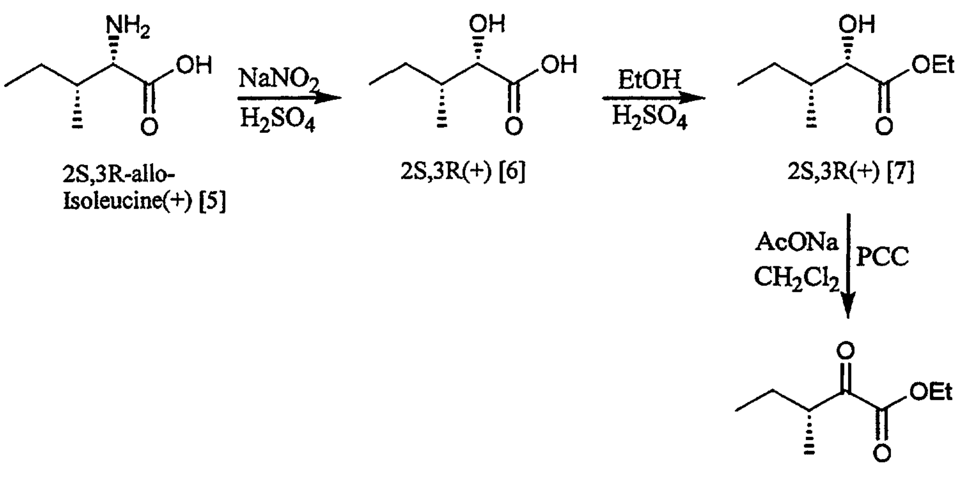


2S,3R-allo-Isoleucine(+) [5]
2S,3R(+) [6]
2S,3R(+) [7]
[8]

*2.1. Synthèse de l'acide (+)-(2S, 3R)-2-hydroxy-3-méthylpentanoique[6]*

On a synthétisé ce composé à partir de (+)-L-allo isoleucine [5] ($[\alpha]^D_{20}$=+37° (5 dans HCl 6M)) comme décrit au paragraphe 1.1 (rendement: 93%).

Données analytiques :

$[\alpha]^D_{20}$ = +18,7° (0,46% dans $CHCl_3$)
SM : 132($M^+$,0) : m/e : 87(36), 76(100), 69(15), 57(28), 45(31), 29(19)
$^1$H-RMN($CDCl_3$) : 0,89(d, J=7, 3H) ; 0,97(t, J=7, 3H) ; 1,38(m, 1H) ; 1,54(m, 1H) ; 1,80(m, 1H) ; 4,30(d, $J_1$=3)
$^{13}$C-RMN($CDCl_3$) : 2q : 11,8, 13,1 ; 1t : 26,0 ; 2d : 38,3, 72,7 ; 1s : 178,9

*2.2. Synthèse du (+)-(2S, 3R)-2-hydroxy-3-méthylpentanoate d'éthyle [7]*

On a synthétisé ce composé à partir de l'(+)-hydroxy acide [6] comme décrit au paragraphe 1.2. (rendement: 66,4%).

Données analytiques :

$[\alpha]^D_{20}$= + 16,1° (0,91% dans $CHCl_3$)
SM : 160($M^+$,1) : m/e : 104(38), 87(90), 76(70), 69(37), 57(32), 45(100), 29(70)
$^1$H-RMN($CDCl_3$) : 0,82(d, J=7, 3H) ; 0,96(t, J=7, 3H) ; 1,31(t, J=7, 3H) ; 1,34(m, 1H) ; 1,53(m, 1H) ; 1,81(m,1H) ; 2,67(d, J=6, 1H, disparition par addition de $D_2O$) ; 4,18(dd, $J_1$=3, $J_2$=6, 1H, déformation à d, J=3 par addition de $D_2O$) ; 4,26(m, 2H)
$^{13}$C-RMN($CDCl_3$) : 3q : 11,9, 13,1, 14,3 ; 2t : 26,0, 61,6 ; 2d : 38,5, 72,9 ; 1s : 175,4

*2.3. Synthèse du (-)-(R)-3-méthyl-2-oxopentanoate d'éthyle [8]*

On a synthétisé ce composé à partir de l'(+)-hydroxy ester [7] comme décrit au paragraphe 1.3. (rendement: 75%).

Données analytiques:

$[\alpha]^D_{20}$= - 36,1° (1,0% dans $CHCl_3$)
Les SM, $^1$H-RMN and $^{13}$C-RMN sont identiques à celles obtenues pour le (+)-isomère (voir 1.3.).

Exemple 2

Composition parfumante

On a préparé une composition parfumante de base pour un parfum de type fleuri-fougère en utilisant les ingrédients suivants :

| Ingrédients | Parties en poids |
|---|---|
| Acétate de benzyle | 170 |
| Acétate de 3,5,5-triméthylcyclohexanyle | 20 |
| Acétate de tricyclo[5.2.1.0.(2,6)]déc-3-én-8-yle | 20 |
| Citronellol | 40 |
| Ethylvanilline à 10%* | 20 |
| Exolide ® 1) | 140 |
| Heliotropine | 40 |
| Iralia ® 2) | 100 |
| Lilial ® 3) | 100 |
| Linalol | 120 |
| Phényléthanol | 40 |
| Salicyclate d'amyle | 40 |
| Salicylate de benzyle | 120 |
| Total | 970 |

* dans le dipropylène glycol
1) mélange de 1-oxacyclohexadécan-2-one et 1-oxa-(12,13)-cyclohexadécen-2-one ; origine : Firmenich SA, Genève, Suisse
2) mélange d'isomères de méthylionones ; origine : Firmenich SA, Genève, Suisse
3) 3-(4-tert-butylphényl)-2-méthylpropanal ; origine : Givaudan-Roure SA, Vernier, Suisse

Lorsqu'on a ajouté à cette composition de base 30 parties en poids de 3-méthyl-2-oxopentanoate d'éthyle ou l'un de ses isomères, on lui a conféré une superbe connotation fruitée avec une légère sous-note de noix très naturelle. On a ainsi conféré à la composition un aspect pétillant très naturel qui a été très apprécié.

Exemple 3

Composition parfumante

On a préparé une composition de base pour un parfum de type rosé-hespéridé à partir des ingrédients suivants :

| Ingrédients | Parties en poids |
|---|---|
| Acétate de diméthylbenzylcarbinyle | 20 |
| Acétate de phényléthyle | 30 |
| Cétone framboise à 10% * | 5 |
| Cétalox®1) à 10% ** | 20 |
| Citronellol | 40 |
| Coumarine | 20 |
| Eugénol | 5 |
| Fructone ®2) | 10 |
| Habanolide ®3) | 120 |
| Hédione®4) | 100 |
| Iralia®5) | 60 |
| Lilial ®6) | 35 |
| Lorysia® 7) | 80 |
| Phénylhexanol | 130 |
| Salicylate d'hexyle | 60 |
| Tétrahydrolinalol | 120 |
| Verdox®8) | 30 |
| Vertofix Coeur 9) | 80 |
| Tamarine Base10) | 35 |
| Total | 1000 |

* dans le dipropylène glycol
* * dans le 2-(2-éthoxyéthoxy)-1-éthanol ; origine : Firmenich SA, Genève, Suisse
1) 8,12-époxy-13,14,15,16-tétranorlabdane ; origine: Firmenich SA, Genève, Suisse
2) 2-méthyl-1,3-dioxolane-2-acétate d'éthyle; origine : International Flavors & Fragrances, USA
3) pentadécénolide ; origine : Firmenich SA, Genève, Suisse
4) dihydrojasmonate de méthyle ; origine : Firmenich SA, Genève, Suisse
5) voir exemple 1
6) voir exemple 1
7) acétate de 4-(1,1-diméthyléthyl)-1-cyclohexyle ; origine : Firmenich SA, Genève, Suisse
8) acétate de 2-tert-butyl-1-cyclohexyle ; origine: International Flavors & Fragrances, USA
9) origine : International Flavors & Fragrances, USA
10) composition à base de limonène ; origine : Firmenich SA, Genève, Suisse

A cette composition de base florale, on a ajouté 30 parties en poids de 3-méthyl-2-oxopentanoate d'éthyle ou l'un de ses isomères. On a ainsi obtenu une composition nouvelle ayant une connotation plus fraîche et plus montante de par la note fruitée agréable que la composition a ainsi acquise.

## Revendications

**1.** Utilisation en tant qu'ingrédient parfumant du 3-méthyl-2-oxopentanoate d'éthyle de formule

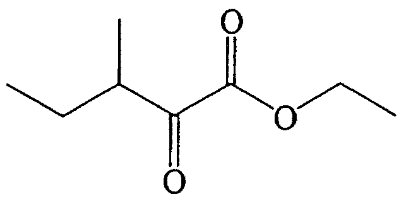

sous la forme d'un isomère optiquement actif ou d'un mélange d'isomères.

2. Composition parfumante ou produit parfumé contenant le 3-méthyl-2-oxopentanoate d'éthyle, tel que définis à la revendication 1, en tant qu'ingrédient parfumant, en mélange avec d'autres ingrédients parfumants, des solvants ou adjuvants courants en parfumerie.

3. Produit parfumé selon la revendication 2, sous forme d'une lotion après-rasage, d'une préparation cosmétique, d'un savon, d'un shampoing ou après-shampoing ou autre produit de soin capillaire, d'un gel de bain ou douche, d'un déodorant corporel ou d'un désodorisant ambiant, d'un détergent ou adoucissant textile, ou d'un produit d'entretien.

4. Produit parfumé selon la revendication 2, sous forme d'un parfum ou d'une eau de toilette.

5. 3-Méthyl-2-oxopentanoate d'éthyle sous forme d'un isomère optiquement actif choisi parmi le groupe comprenant le (+)-(S)-3-méthyl-2-oxopentanoate d'éthyle et le (-)-(R)-3-méthyl-2-oxopentanoate d'éthyle.

6. Procédé pour la préparation du 3-méthyl-2-oxopentanoate d'éthyle selon la revendication 5, **caractérisé en ce que** le (+)-(2S,3S)-2-hydroxy-3-méthyl-pentanoate d'éthyle, respectivement (+)-(2S, 3R)-2-hydroxy-3-méthyl-pentanoate d'éthyle, est oxydé à l'aide d'acétate de sodium et de chlorochromate de pyridine successivement.

## Claims

1. Use as perfuming ingredient of ethyl 3-methyl-2-oxopentanoate, of formula

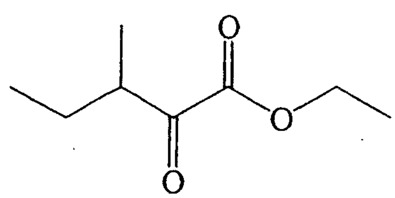

in the form of an optically active isomer or of a mixture thereof.

2. A perfuming composition or a perfumed product containing ethyl 3-methyl-2-oxopentanoate, as defined in claim 1, as perfuming ingredient, in admixture with other perfuming ingredients, solvents or adjuvants of current use in perfumery.

3. Perfumed product according to claim 2, in the form of an after-shave lotion, a cosmetic preparation, a soap, a shampoo or hair-conditioner or another hair-care product, a bath or shower gel, a body or air deodorant, a detergent or a fabric softener, or a household product.

4. Perfumed product according to claim 2, in the form of a perfume or a cologne.

5. 3-Methyl-2-oxo-ethyl-pentanoate in the form of an optically active isomer, selected from the group consisting of (+)-ethyl-(S)-3-methyl-2-oxopentanoate and (-)-ethyl-(R)-3-methyl-2-oxopentanoate.

6. Process for the preparation of 3-methyl-2-oxo-ethyl-pentanoate according to claim 5, **characterized in that** (+)-ethyl-(2S, 3S)-2-hydroxy-3-methylpentanoate, respectively (+)-ethyl-(2S, 3R)-2-hydroxy-3-methylpentanoate is oxidized by means of successively sodium acetate and pyridinium chlorochromate.

**Patentansprüche**

1. Verwendung von 3-Methyl-2-oxopentansäureethylester der Formel

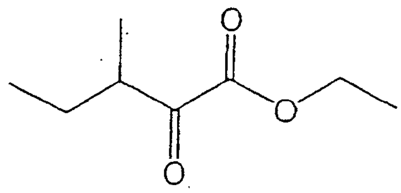

in Form eines optisch aktiven Isomeren oder einer Isomerenmischung als Duftstoff.

2. Duftzusammensetzung oder parfümiertes Produkt, enthaltend den wie in Anspruch 1 definierten 3-Methyl-2-oxopentansäureethylester als Duftstoff in Mischung mit anderen Duftstoffen, Lösungsmitteln oder in der Kosmetikindustrie gängigen Hilfsstoffen.

3. Parfümiertes Produkt nach Anspruch 2 in Form einer After-Shave-Lotion, eines Kosmetikpräparats, einer Seife, eines Shampoos oder After-Shampoos oder eines anderen Haarpflegeprodukts, eines Bade- oder Duschgels, eines Körperdeodorants oder eines Raumlufterfrischers, eines Waschmittels oder Gewebeweichspülers oder eines Pflegemittels.

4. Parfümiertes Produkt nach Anspruch 2 in Form eines Parfüms oder eines Eau de Toilette.

5. 3-Methyl-2-oxopentansäureethylester in Form eines optisch aktiven Isomeren, ausgewählt aus der Gruppe umfassend (+)-(S)-3-Methyl-2-oxopentansäureethylester und (-) - (R)-3-Methyl-2-oxopentansäureethylester.

6. Verfahren zur Herstellung von 3-Methyl-2-oxopentansäureethylester nach Anspruch 5, **dadurch gekennzeichnet, daß** (+)-(2S,3S)-2-Hydroxy-3-methylpentansäureethylester bzw. (+)-(2S,3R)-2-Hydroxy-3-methylpentansäureethylester nacheinander mit Natriumacetat und Pyridiniumchlorochromat oxidiert werden.